# EUROPEAN PATENT APPLICATION

(11) **EP 4 241 581 A1**
(43) Date of publication of application: **13.09.2023**
(21) Application number: 21889221.4
(22) Date of filing: 04.11.2021
(51) Int. Cl.: A23P 10/00, A23P 30/00

(54) **FOOD PROCESSING DEVICE AND REACTION TUBE WITH LIGHT SOURCE**

(30) Priority: 04.11.2020 JP 2020184201
(71) Applicant: Panasonic Intellectual Property Management Co., Ltd., Osaka-shi, Osaka 540-6207 (JP)
(72) Inventor: UKAI, Kunihiro, Osaka-shi, Osaka 540-6207 (JP); HASHIMOTO, Yasuhiro, Osaka-shi, Osaka 540-6207 (JP); INO, Daisuke, Osaka-shi, Osaka 540-6207 (JP)
(74) Representative: Eisenführ Speiser
(86) International application number: PCT/JP2021/040529
(87) International publication number: WO 2022/097668

(57) **Abstract**

A food processing apparatus (100) includes a reaction tank (1) having a first space (S 1) for storing a liquid reactant to be used for a food; a stirrer (2) including a first stirring body (4) that stirs the reactant in the reaction tank (1) by rotating; catalytic reactors (6), the catalytic reactors (6) each including a reaction tube (7) and a light source (8) disposed in the reaction tube (7), the reaction tube (7) having an outer surface provided with a photocatalyst, the reaction tube (7) allowing light emitted from the light source (8) to pass therethrough; and a temperature adjuster (10) that adjusts the temperature of the reactant in the reaction tank (1). The catalytic reactors (6) are disposed around a first rotary shaft (3) of the first stirring body (4) to be spaced from each other. The temperature adjuster (10) surrounds the catalytic reactors (6).

## Description

### Technical Field

The present disclosure relates to a food processing apparatus and a light-source-equipped reaction tube.

### Background Art

PTL 1 discloses a manufacturing method in which microorganisms in a brewed product are sterilized under a non-heated normal temperature by using a photocatalyst in a process of manufacturing a food.

### Citation List

### Patent Literature

PTL 1: Japanese Unexamined Patent Application Publication No. 2003-250514

### Summary of Invention

There is, however, room for improvement in the apparatus or the manufacturing method in PTL 1 mentioned above. For example, there is a problem that it is difficult to effectively reform a reactant that is newly used for a food.

One aspect of the present disclosure has been made in consideration of such a circumstance and provides a food processing apparatus capable of effectively reforming a reactant that is newly used for a food.

A food processing apparatus according to one aspect of the present disclosure includes a reaction tank that has a first space for storing a liquid reactant that is to be used for a food; a stirrer that includes a first stirring body that stirs the reactant in the reaction tank by rotating; catalytic reactors, the catalytic reactors each including a reaction tube and a light source disposed in the reaction tube, the reaction tube having an outer surface provided with a photocatalyst, the reaction tube allowing light that is emitted from the light source to pass through the reaction tube; and a temperature adjuster that adjusts a temperature of the reactant in the reaction tank, in which the catalytic reactors are disposed around a first rotary shaft of the first stirring body to be spaced from each other, and in which the temperature adjuster surrounds the catalytic reactors.

This comprehensive or specific aspect may be realized by a method or a system and may be realized by any selective combination of an apparatus, a method, a system, and a method.

The food processing apparatus according to one aspect of the present disclosure can be operated stably and can effectively reform a reactant that is used for a food.

### Brief Description of Drawings

[Fig. 1] Fig. 1 illustrates one example of a food processing apparatus in an embodiment.
[Fig. 2A] Fig. 2A illustrates one example of a specific configuration of a stirring body in an embodiment.
[Fig. 2B] Fig. 2B illustrates another example of a specific configuration of a stirring body in an embodiment.
[Fig. 2C] Fig. 2C illustrates another example of a specific configuration of a stirring body in an embodiment.
[Fig. 2D] Fig. 2D illustrates another example of a specific configuration of a stirring body in an embodiment.
[Fig. 3] Fig. 3 illustrates one example of a configuration of a catalytic reactor according to an embodiment.
[Fig. 4] Fig. 4 illustrates one example of a case where a catalytic reactor according to an embodiment is fixed to a lid of a reaction tank 1.
[Fig. 5] Fig. 5 illustrates one example of a case where a catalytic reactor according to an embodiment is fixed to the bottom of the reaction tank 1.
[Fig. 6] Fig. 6 is a VI-VI sectional view of the food processing apparatus in Fig. 1 according to an embodiment.
[Fig. 7] Fig. 7 schematically illustrates a flow of a reactant in a food processing apparatus according to an embodiment.
[Fig. 8] Fig. 8 illustrates one example of a configuration of a stirrer of a food processing apparatus according to Modification 1.
[Fig. 9] Fig. 9 illustrates one example of an installation configuration of catalytic reactors of a food processing apparatus according to Modification 2.
[Fig. 10] Fig. 10 illustrates one example of a food processing apparatus according to Modification 3.
[Fig. 11] Fig. 11 illustrates one example of a food processing apparatus according to Modification 4.
[Fig. 12] Fig. 12 illustrates one example of a food processing apparatus according to Modification 5.
[Fig. 13] Fig. 13 illustrates one example of a food processing apparatus according to Modification 6.

### Description of Embodiments

### (Underlying Knowledge Forming Basis of the Present Disclosure)

The inventors of the present invention have found that the following problems occur in the food manufacturing apparatus or the food manufacturing method described in the section of the "Background Art".

In manufacture of a food, it is widely performed for the purpose of, for example, improving efficiency of the manufacture or increasing the content of a nutritional component to reform a raw material used for the food.

As a method of reforming a raw material of a food, there is a method that uses a catalyst. For example, there is a method of using a nickel catalyst to hydrogenate an oil/fat component serving as a raw material in manufacture of margarine. Using an immobilized enzyme in manufacture of a food also can be one usage of a catalyst.

Although not from the point of view of reforming a raw material of a food, a catalyst may be used for the purpose of sterilization in a manufacturing process. For example, in PTL 1, a manufacturing method in which a photocatalyst is used in a process of manufacturing a food to sterilize microorganisms in a brewed product under a non-heated normal temperature is examined.

Although being effective for reformation of a single-component raw material as a raw material used for a food, known methods using catalysts are extension of a chemical engineering method and thus have a limitation in apparatus configurations, and, in order to cope with configurations suitable for catalysts used for development to multiple purposes, there is room for improvement.

Since apparatuses used in manufacturing methods using a photocatalyst are also used for the purpose of sterilization, there is room for improvement to cope with configurations suitable for reforming raw materials of foods.

To solve the aforementioned problems, a food processing apparatus according to one aspect of the present disclosure includes a reaction tank that has a first space for storing a liquid reactant that is to be used for a food; a stirrer that includes a first stirring body that stirs the reactant in the reaction tank by rotating; catalytic reactors, the catalytic reactors each including a reaction tube and a light source disposed in the reaction tube, the reaction tube having an outer surface provided with a photocatalyst, the reaction tube allowing light that is emitted from the light source to pass through the reaction tube; and a temperature adjuster that adjusts a temperature of the reactant in the reaction tank, in which the catalytic reactors are disposed around a first rotary shaft of the first stirring body to be spaced from each other, and in which the temperature adjuster surrounds the catalytic reactors.

According to this, the light source emits the light from the inner side of the reaction tube, and thus, a thin film of the photocatalyst of the reaction tube can be irradiated with the light of the light source directly, without through the reactant. Therefore, it is possible to cause generation of an exciton of the photocatalyst to be not easily inhibited and thus is possible to improve reactivity of the raw material with respect to the photocatalyst. Since the reactant after a reaction is immediately sent by the stirrer to the temperature adjuster disposed outside the reaction tubes, it is possible to adjust the temperature of the reactant to an appropriate temperature and possible to effectively suppress a temperature increase of the reactant in the vicinity of the reaction tubes due to the heat generated by the light sources in the reaction tubes. Since, as described above, the temperature of the reactant can be effectively adjusted to an intended temperature, reactivity of the reactant is easily managed. In other words, a food processing apparatus can be realized with a simple configuration, and the food processing apparatus can be stably operated and exerts an effect of enabling effective reformation of a reactant that used for a food.

The temperature adjuster may include an outer wall that surrounds the reaction tank, and a heating medium that circulates in a second space between the reaction tank and the outer wall.

Consequently, the temperature of the reactant in the reaction tank can be effectively adjusted.

The heating medium may be a refrigerant that cools the reactant.

Consequently, it is possible to adjust the reactant in the reaction layer to have a target temperature.

A temperature detector that is disposed in the reaction tank and detects a temperature of the reactant may be further included, and the temperature adjuster may operate based on the temperature detected by the temperature detector to thereby adjust the temperature of the reactant.

Consequently, it is possible to effectively cool the reactant in the reaction layer.

The first stirring body may include at least one of a propeller blade, a disk turbine blade, an inclined paddle blade, and a centrifugal stirring body.

Consequently, it is possible to effectively stir the reactant in the reaction layer.

The stirrer may include a second stirring body, and the first rotary shaft and the second stirring body may have an identical rotational axis.

Consequently, it is possible to shorten the circulation path of the reactant in the reaction layer and possible to effectively stir the reactant.

A stirring plate that protrudes from an inner wall surface of the reaction tank toward an inner side of the reaction tank and that is disposed along the first rotary shaft may be further included.

Consequently, it is possible to effectively stir the reactant in the reaction layer. Since the stirring plate can be used as a fin that accelerates heat conduction between the reactant and the temperature adjuster, heat exchange between the reactant and the temperature adjuster can be effectively performed.

The catalytic reactors may include first catalytic reactors that are located in a first region and second catalytic reactors that are located in a second region, the first region may be a region between a region whose distance from the first rotary shaft is a first distance and a region whose distance from the first rotary shaft is a second distance, the second region may be a region between a region whose distance from the first rotary shaft is a third distance and a region whose distance from the first rotary shaft is a fourth distance, the first distance may be smaller than the second distance, the second distance may be smaller than the third distance, and the third distance may be smaller than the fourth distance.

Therefore, the reactant that has passed among the first catalytic reactors easily comes into contact with the second catalytic reactors disposed on the outer side thereof. It is thus possible to improve the reactivity of the reactant with respect to the catalytic reactors.

The reaction tube may include a bottomed cylindrical glass substrate and a thin film of a photocatalyst provided on an outer surface of the glass substrate, and the glass substrate may be disposed in an orientation in which a cylinder axis direction of the glass substrate is along the first rotary shaft.

A lid that covers an upper portion of the reaction tank may be further included, and the lid may include a lid-side fixing member that fixes the catalytic reactors.

Therefore, the catalytic reactors can be fixed to the lid.

A bottom-side fixing member that is disposed at a bottom of the reaction tank and fixes the catalytic reactors to the bottom may be further included.

Therefore, the catalytic reactors can be fixed to the bottom of the reaction tank.

A light-source-equipped reaction tube according to one aspect of the present disclosure is a light-source-equipped reaction tube that is to be used for a catalytic reactor of the aforementioned food processing apparatus, the light-source-equipped reaction tube including a reaction tube that has an outer surface provided with a photocatalyst and that allows light to pass through the reaction tube, and a light source that emits light from an inner side of the reaction tube.

According to this, the light source emits the light from the inner side of the reaction tube, and thus, a thin film of the photocatalyst of the reaction tube can be irradiated with the light of the light source directly, without through the reactant. Therefore, it is possible to cause generation of an exciton of the photocatalyst to be not easily inhibited and thus is possible to improve reactivity of the raw material with respect to the photocatalyst.

Hereinafter, specific examples of an embodiment will be described with reference to the accompanying drawings.

Each of the specific examples described below is one example of the aforementioned aspects. Accordingly, the shapes, materials, components, arrangement positions and connection forms of the components, and the like presented below are not intended to limit the aforementioned aspects unless those are described in the claims. Among the following components, components that are not described in the independent claims defining the most generic concept of the present aspects are described as optional components. Description of components denoted by the same reference signs in the drawings may be omitted. In the drawings, each component is schematically illustrated for ease of understanding, and shapes, dimensional ratios, and the like thereof may not be accurately illustrated.

### (Embodiment)

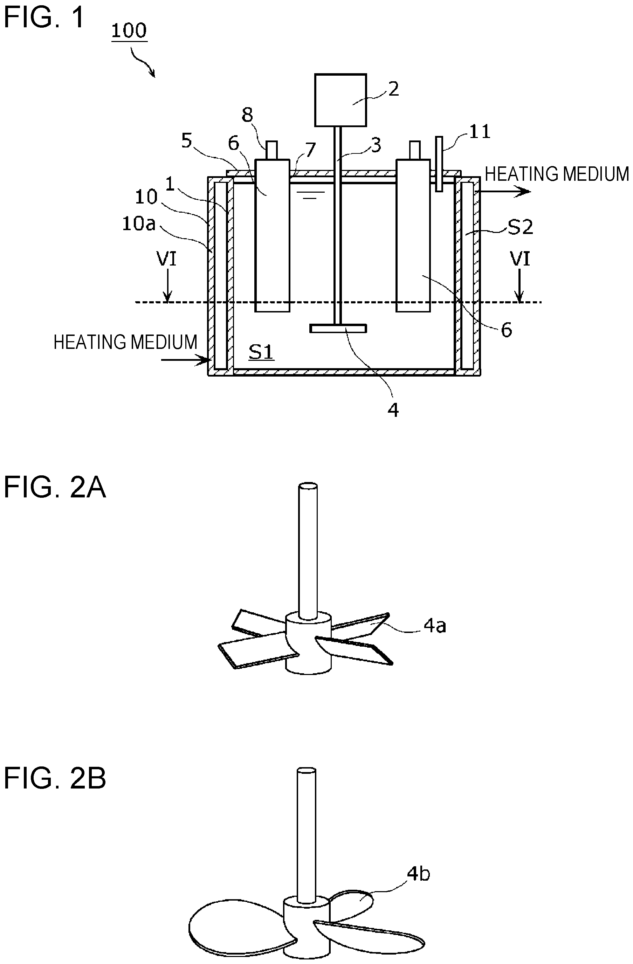

A configuration of a food processing apparatus 100 will be described with Fig. 1. Fig. 1 illustrates one example of the food processing apparatus 100 in an embodiment.

As illustrated in Fig. 1, the food processing apparatus 100 includes a reaction tank 1, a stirrer 2, catalytic reactors 6, and a temperature adjuster 10.

The reaction tank 1 has a first space S1 for storing a liquid reactant that is to be used for a food. The reaction tank 1 is, for example, a bottomed cylindrical container. The reaction tank 1 does not necessarily have a cylindrical shape as long as the reaction tank 1 is a bottomed tubular container having the first space S1 for storing a liquid reactant. The reaction tank 1 is provided with a lid 5 that closes an opening at an upper portion of the reaction tank 1. The lid 5 is a disk-shaped member and has through holes through which a rotary shaft 3 of a stirring body 4, the catalytic reactors 6, and a temperature detector 11 pass.

The stirrer 2 includes the stirring body 4 that rotates to thereby stir the reactant in the reaction tank 1. The stirrer 2 is disposed such that the rotary shaft 3 of the stirrer 2 corresponds to the center axis of the cylinder of the reaction tank 1. The stirrer 2 includes a motor, not illustrated, that rotates the rotary shaft 3.

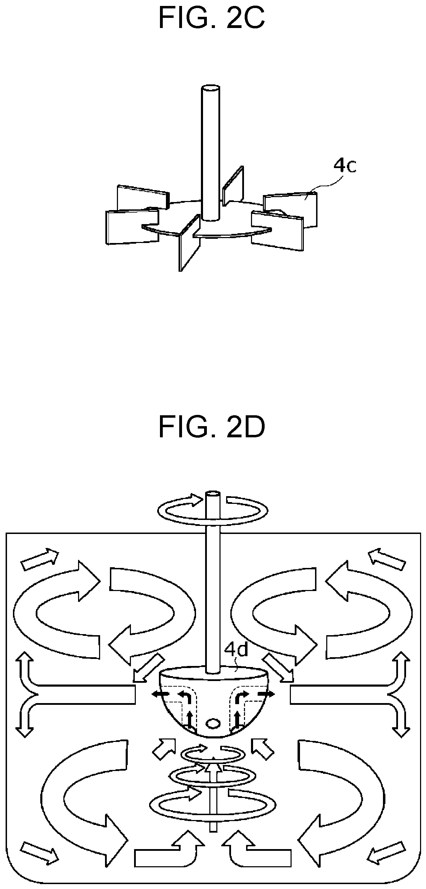

Here, a specific example of the stirring body 4 will be described with Fig. 2A to Fig. 2D. Fig. 2A to Fig. 2D each illustrate one example of a specific configuration of the stirring body 4.

As illustrated in Fig. 2A, the stirring body 4 may be realized by, for example, an inclined paddle blade 4a. The stirring body 4 may be realized by any one of a propeller blade 4b, a disk turbine blade 4c, and a centrifugal stirring body 4d respectively illustrated in Fig. 2B to Fig. 2D to achieve optimal processing conditions in consideration of viscosity of the reactant and operation processing conditions such as power consumption of the stirrer 2. When stirring bodies 4 are used in the food processing apparatus 100, it is sufficient for each stirring body 4 to include at least one of the inclined paddle blade 4a, the propeller blade 4b, the disk turbine blade 4c, and the centrifugal stirring body 4d.

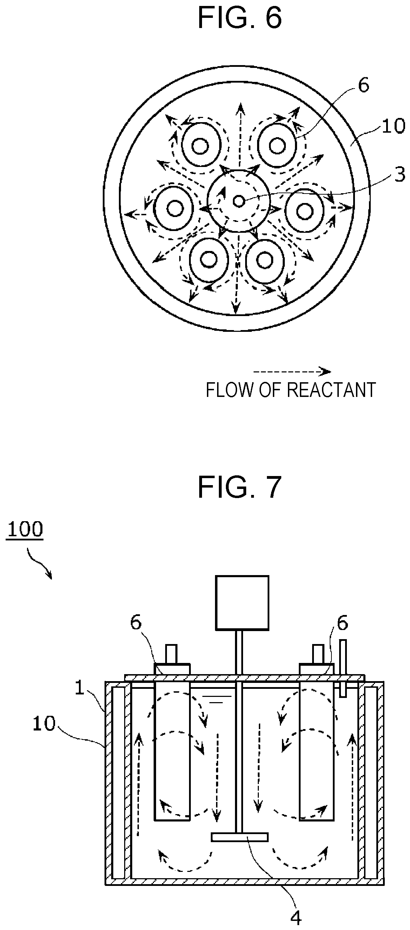

As illustrated in Fig. 6 mentioned later, the (six in the present embodiment) catalytic reactors 6 are disposed around the rotary shaft 3 of the stirring body 4 in a state of being spaced from each other when viewed in the axial direction of the rotary shaft 3. As a result, the outer side of the six catalytic reactors 6 is surrounded by the inner wall surface of the reaction tank 1. Consequently, when the reactant inside the reaction tank 1 is stirred by the stirrer 2, the stirred reactant can move among the catalytic reactors 6.

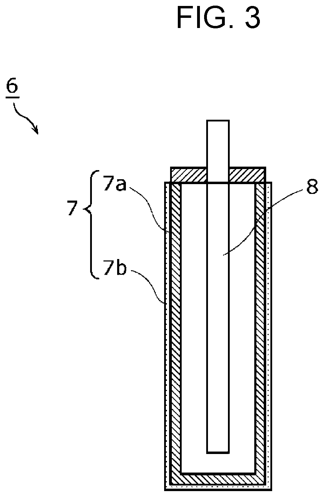

Here, details of the configuration of the catalytic reactors 6 will be described with Fig. 3. Fig. 3 illustrates one example of the configuration of the catalytic reactors 6 according to the present embodiment.

As illustrated in Fig. 3, each of the catalytic reactors 6 includes a reaction tube 7 that has an outer surface provided with a photocatalyst and that allows light to pass therethrough and a light source 8 that emits light from the inner side of the reaction tube 7. In other words, each catalytic reactor 6 is a light-source-equipped reaction tube including the reaction tube 7 and the light source 8. Specifically, the reaction tube 7 includes a glass substrate 7a having a bottomed cylindrical shape, and a thin film 7b of the photocatalyst provided on the outer surface of the glass substrate 7a. The glass substrate 7a is disposed such that the cylinder axis direction of the cylindrical shape of the glass substrate 7a extends along the rotary shaft 3 of the stirring body 4. The light source 8 is inserted into the inside of the glass substrate 7a from an open portion of the glass substrate 7a opposite to the bottom thereof.

The thin film 7b of the photocatalyst provided on the outer surface of the glass substrate 7a is formed by, for example, a general sol-gel method. Specifically, the thin film 7b of the photocatalyst is constituted by TiO₂. A sol-gel solution used in the method of forming the thin film 7b of the photocatalyst is applied to the outer surface of the glass substrate 7a, and the glass substrate 7a with the sol-gel solution applied thereto is rotated by using a rotary machine. Consequently, the sol-gel solution is uniformly applied to the entirety of the outer surface of the glass substrate 7a. After the sol-gel solution is dried, the glass substrate 7a on which the sol-gel solution is applied is heated, after dried in an electric furnace, at a high temperature of higher than or equal to 500°C, and the thin film 7b of the photocatalyst is thereby heat-treated on the outer surface of the glass substrate 7a.

Specifically, the light source 8 includes a light source having a center wavelength of approximately 260 nm to 400 nm to effectively generate an exciton of the photocatalyst. In the present embodiment, the light source 8 includes an LED (Light Emitting Diode) having a wavelength of 365 nm as the center wavelength. The light source 8 may be disposed to face the thin film 7b inside the reaction tube 7 so that the thin film 7b provided on the outer surface of the glass substrate 7a is effectively irradiated with light. For example, the light source 8 is constituted by LEDs that are disposed side by side in the longitudinal direction of a rod-shaped member that serves as a substrate. The LEDs disposed side by side in the longitudinal direction may be disposed such that, for example, rows of the LEDs are disposed side by side around the rod-shaped member in the axial direction. Consequently, the light source 8 can irradiate the entirety of the thin film 7b of the cylindrical reaction tube 7 with light. The light source 8 is not limited to an LED and may be, for example, a high-pressure mercury lamp, a low-pressure mercury lamp, or a fluorescent light-type light source containing a fluorescent agent.

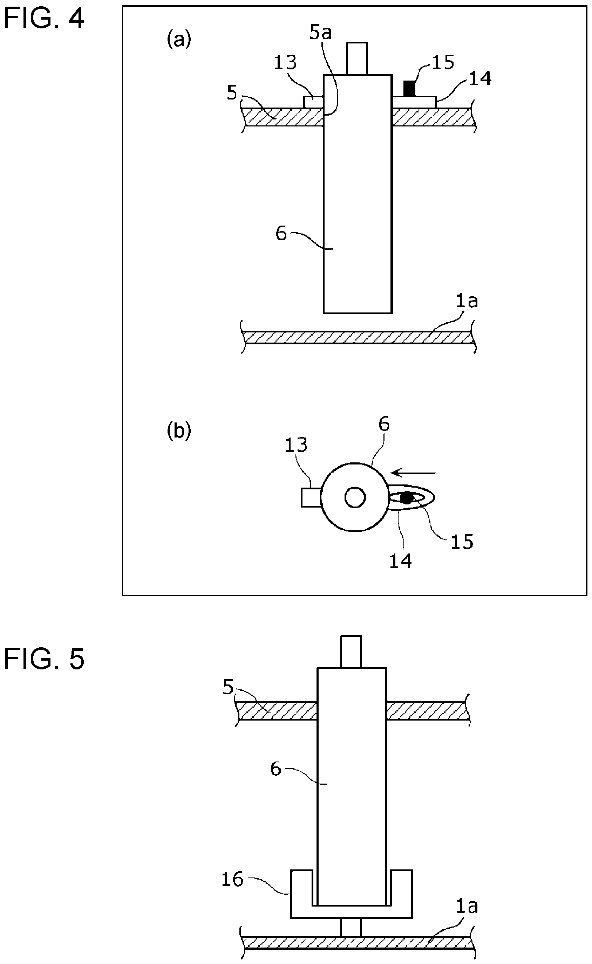

Next, a form of fixing the catalytic reactors 6 to the reaction tank 1 will be described with Fig. 4 and Fig. 5. Fig. 4 illustrates one example of a case where the catalytic reactors 6 according to the present embodiment are fixed to the lid 5 of the reaction tank 1. Fig. 5 illustrates one example of a case where the catalytic reactors 6 according to the present embodiment are fixed to the bottom of the reaction tank 1.

As illustrated in Fig. 4, the catalytic reactors 6 each may pass through a corresponding one of through holes 5a provided in the lid 5 of the reaction tank 1 and may be fixed at the lid 5 to the reaction tank 1. In this case, a pressing portion 13 and a movable pressing portion 14 that is movable in an arrangement direction of the pressing portion 13 and the movable pressing portion 14 are disposed on the lid 5 such that the through hole 5a through which the catalytic reactor 6 passes is interposed between the pressing portion 13 and the movable pressing portion 14. In a state in which the catalytic reactor 6 is held by the pressing portion 13 and the movable pressing portion 14, movement of the movable pressing portion 14 is fixed by a pressing screw 15. As described above, by being fixed to the lid 5, the catalytic reactor 6 can mitigate an influence of the catalytic reactor 6 being displaced by the flow of the reactant due to stirring. The pressing portion 13, the movable pressing portion 14, and the pressing screw 15 are one example of a lid-side fixing member. Each catalytic reactor 6 can be detached from the lid 5 by loosening the pressing screw 15 to cause the movable pressing portion 14 to be in a movable state. In other words, each catalytic reactor 6 is attachable to and detachable from the reaction tank 1.

Further, as illustrated in Fig. 5, the catalytic reactors 6 may be each fixed by a bottom-side fixing member 16 provided on a bottom 1a of the reaction tank 1 to the reaction tank 1 at the bottom 1a. For example, the bottom-side fixing member 16 is disposed on the bottom 1a of the reaction tank 1, includes an upper portion having a recessed shape on the upper side, and supports the bottom of the catalytic reactor 6 by the recessed shape. Consequently, the bottom-side fixing member 16 can fix the bottom of the catalytic reactor 6 to the bottom 1a of the reaction tank 1 and can mitigate an influence of the catalytic reactor 6 being displaced by the flow of the reactant due to stirring.

Each of the catalytic reactors 6 may be fixed as illustrated in Fig. 4 by the lid-side fixing member of the lid 5, may be fixed as illustrated in Fig. 5 at the bottom 1a by the bottom-side fixing member 16, and may be fixed by both the lid-side fixing member and the bottom-side fixing member 16.

The temperature adjuster 10 adjusts the temperature of the reactant in the reaction tank 1. The temperature adjuster 10 is disposed to surround the outer side of the catalytic reactors 6. Specifically, the temperature adjuster 10 includes an outer wall 10a that surrounds the reaction tank 1, and a heating medium that circulates in the second space S2 between the reaction tank 1 and the outer wall 10a.

The temperature adjuster 10 adjusts the temperature of the reactant by operating on the basis of the temperature detected by the temperature detector 11. Specifically, when the reactant having a temperature higher than a first temperature is to be cooled to the first temperature, the temperature adjuster 10 causes, as the heating medium, a refrigerant having a temperature lower than or equal to the first temperature to circulate in the second space S2. Consequently, the temperature adjuster 10 cools the reactant by causing the refrigerant and the reactant to exchange heat therebetween with the reaction tank 1 interposed between the refrigerant and the reactant. For example, the second space S2 may be connected by a pipe, not illustrated, to a heat exchanger, not illustrated, disposed outside the second space S2 so that the refrigerant whose temperature has increased as a result of heat exchange with the reactant is cooled in the heat exchanger to a temperature lower than or equal to the first temperature and then returns to the second space S2. The refrigerant may be circulated between the second space S2 and the aforementioned heat exchanger by, for example, a circulation pump, not illustrated. In this case, the temperature adjuster 10 may start cooling of the reactant by starting operation of the circulation pump.

The temperature adjuster 10 may heat the reactant having a temperature lower than a second temperature to the second temperature. In this case, the temperature adjuster 10 causes a heating medium having a temperature higher than or equal to the second temperature to circulate in the second space S2. Consequently, the temperature adjuster 10 heats the reactant by causing the heating medium and the reactant to exchange heat therebetween with the reaction tank 1 interposed between the heating medium and the reactant. For example, the second space S2 may be connected by a pipe, not illustrated, to a heat exchanger, not illustrated, disposed outside the second space S2 so that the heating medium whose temperature has decreased as a result of heat exchange with the reactant is heated to a temperature higher than or equal to the second temperature in the heat exchanger and then returns to the second space S2.

The temperature detector 11 is disposed inside the reaction tank 1 and detects the temperature of the reactant. The temperature detector 11 is constituted by, for example, a thermistor, a thermocouple, or the like. The temperature detector 11 passes through the lid 5 and, for example, is fixed to the lid 5.

Next, the operation of the food processing apparatus 100 will be described with Fig. 1, Fig. 6, and Fig. 7. Fig. 6 is a VI-VI sectional view of the food processing apparatus 100 in Fig. 1 according to an embodiment. The figure schematically illustrates a flow of the reactant in the food processing apparatus 100 according to an embodiment.

First, in the food processing apparatus 100, a reactant serving as a raw material of a food is input into the reaction tank 1. Next, the food processing apparatus 100 starts light emission to the thin film 7b of the photocatalyst from the inside of the reaction tube 7 by turning on the light sources 8 of the catalytic reactors 6. The food processing apparatus 100 rotates the rotary shaft 3 of the stirring body 4 by driving the motor of the stirrer 2 and stirs the reactant in the reaction tank 1. In addition, the food processing apparatus 100 supplies a heating medium to the second space S2 of the temperature adjuster 10 by driving a circulation pump of the temperature adjuster 10.

At this time, the food processing apparatus 100 detects the temperature of the reactant by the temperature detector 11 and adjusts the temperature of the heating medium that is to be supplied to the second space and/or the supply amount of the heating medium to cause the reactant to have a preset temperature. The food processing apparatus 100 adjusts the temperature of the heating medium by, for example, adjusting the amount of heat exchange in the heat exchanger installed outside the second space S2. Specifically, the food processing apparatus 100 may adjust the temperature of the heating medium by adjusting, when the heat exchanger performs air-cooling, the air volume of a fan that accelerates the air-cooling in the heat exchanger and may adjust the temperature of the heating medium by adjusting, when the heat exchanger performs water-cooling, the water volume of a pump that accelerates the water-cooling in the heat exchanger. The food processing apparatus 100 may adjust the supply amount of the heating medium supplied to the second space by adjusting the amount of circulation by the circulation pump for causing the heating medium to circulate between the second space S2 outside the reaction tank 1 and the heat exchanger. As described above, the temperature and the supply amount of the heating medium can be adjusted by using a constant-temperature water circulation apparatus (not illustrated) or the like including a heat exchanger, a circulation pump, and a pipe.

For example, when the reaction of the reactant in the food processing apparatus 100 is fermentation such as that of yogurt, a temperature (approximately 40°C for lactic acid bacteria) suitable for the fermentation may be maintained to prevent propagation of unwanted bacteria. In this case, the temperature that is preset as a target in the temperature adjuster 10 is 40°C. When the reaction of the reactant in the food processing apparatus 100 is fermentation of beer yeast, the beer yeast may be fermented at a low temperature (for example, approximately 5°C). In this case, the temperature that is preset as a target in the temperature adjuster 10 is 5°C.

In the food processing apparatus 100, the photocatalyst irradiated with light and the reactant serving as a raw material of a food are brought into contact with each other to reform the reactant by the photocatalyst. For example, in a case where a raw material of yogurt is reformed, the protein in the raw material can be decomposed by the photocatalyst and can have a mild flavor. In a case where a raw material of beer is reformed, the period of time of fermentation can be shortened by previously decomposing the sugars in wort.

However, the temperature of the reactant in the vicinity of the catalytic reactors 6 is increased by being influenced by the heat generated by the light sources 8. Meanwhile, the food processing apparatus 100 is required to perform temperature management of the reactant strictly to thereby maintain the quality of a food or the like generated by a reaction to a level that is higher than or equal to a certain level. Therefore, it is important in the food processing apparatus 100 that both a reaction with the photocatalyst and temperature adjustment of the reactant are achieved.

Thus, in the food processing apparatus 100 according to the present embodiment, the catalytic reactors 6 are disposed in a state of being spaced from each other around the rotary shaft 3 of the stirring body 4. Therefore, when the reactant is moved by being stirred, the reactant can move by passing among the catalytic reactors 6. Consequently, even though the reactant in the vicinity of the reaction tube 7 is heated by the heat generated by the light sources 8 in the reaction tube 7, the reactant after caused to pass among the catalytic reactors 6 by the stirrer 2 is smoothly sent to the temperature adjuster 10 disposed outside the reaction tube 7 and is cooled. Since, as described above, the temperature of the reactant can be efficiently adjusted to an intended temperature, the reaction of the reactant is easily managed.

As illustrated in Fig. 6, the reactant in the vicinity of the reaction tubes 4 flows along the side surfaces of the reaction tubes 7 to the outer side due to stirring by the stirring body 4 and flows toward the inner wall surface of the reaction tank 1 after flowing in a space between, of the reaction tubes 7, two mutually adjacent reaction tubes 7. Since, as described above, a space is provided between two mutually adjacent reaction tubes 7, the space between the two reaction tubes 7 is a narrow space and can increase the flow rate of the flow along the reaction tubes 7. Consequently, the boundary film thickness of the thin film 7b of the photocatalyst of the reaction tube 7 at the boundary between the thin film 7b and the reactant can be reduced, and thus, reactivity with respect to the photocatalyst can be improved.

The reactant that flows in the vicinity of the reaction tubes 7 and that is heated by the heat generated from the reaction tubes 7 can be mixed with a reactant that flows in a space between two mutually adjacent reaction tubes 7 and that is less influenced by the generated heat. It is thus possible to reduce the influence of a temperature increase of the reactant due to the heat generated from the reaction tubes 7. Further, as illustrated in Fig. 7, the reactant that has flowed to the inner wall surface of the reaction tank 1 flows along the inner wall surface. It is thus possible to improve performance of heat exchange with the heating medium that flows inside the temperature adjuster 10 and possible to effectively adjust the temperature of the reactant.

As illustrated in Fig. 7, in the configuration of the stirrer 2 according to the present embodiment, the flow of the reactant in an upper portion of the reaction tank 1 is opposite the flow thereof in a bottom portion. In other words, a flow that moves from the outer side toward the inner side of the reaction tank 1 is generated in the upper portion of the reaction tank 1, and a flow that moves from the inner side toward the outer side of the reaction tank 1 is generated in the bottom portion of the reaction tank 1. Since, as described above, the reactant flows in different directions in the upper portion and the bottom portion, the reactant whose temperature has been sufficiently adjusted by the temperature adjuster 10 flows in the upper portion of the reaction tank 1 toward the stirrer 2 by passing between two mutually adjust reaction tubes 7. Also in the upper portion of the reaction tank 1, as in the bottom portion, a space is provided between two mutually adjacent reaction tubes 7, and the space between the two reaction tubes 7 is thus a narrow space and can increase the flow rate of the flow along the reaction tubes 7. Consequently, the boundary film thickness of the thin film 7b of the photocatalyst of the reaction tube 7 can be reduced, and thus, reactivity with respect to the photocatalyst can be improved.

Also in the upper portion of the reaction tank 1, the reactant that flows in the vicinity of the reaction tubes 7 and that is heated by the heat generated from the reaction tubes 7 can be mixed with a reactant that flows in a space between two mutually adjacent reaction tubes 7 and that is less influenced by the generated heat. It is thus possible to reduce the influence of a temperature increase of the reactant due to the heat generated from the reaction tubes 7.

As described above, in the food processing apparatus 100 described in the present embodiment, the light generated by the light source 8 is emitted from the inner side of the reaction tube 7, and it is thus possible to cause the light of the light source 8 to irradiate directly, without through the reactant, the thin film 7b of the photocatalyst of the reaction tube 7. Therefore, it is possible to cause generation of an exciton of the photocatalyst to be not easily inhibited and thus is possible to improve reactivity of the raw material with respect to the photocatalyst. Since the reactant after a reaction is immediately sent by the stirrer 2 to the temperature adjuster 10 disposed outside the reaction tubes 7, it is possible to adjust the temperature of the reactant to an appropriate temperature and possible to effectively suppress a temperature increase of the reactant in the vicinity of the reaction tubes 7 due to the heat generated by the light sources 8 in the reaction tubes 7. Since, as described above, the temperature of the reactant can be effectively adjusted to an intended temperature, reactivity of the reactant is easily managed. In other words, a food processing apparatus can be realized with a simple configuration, and the food processing apparatus can be stably operated and exerts an effect of enabling effective reformation of a reactant that is used for a food.

### (Modification 1)

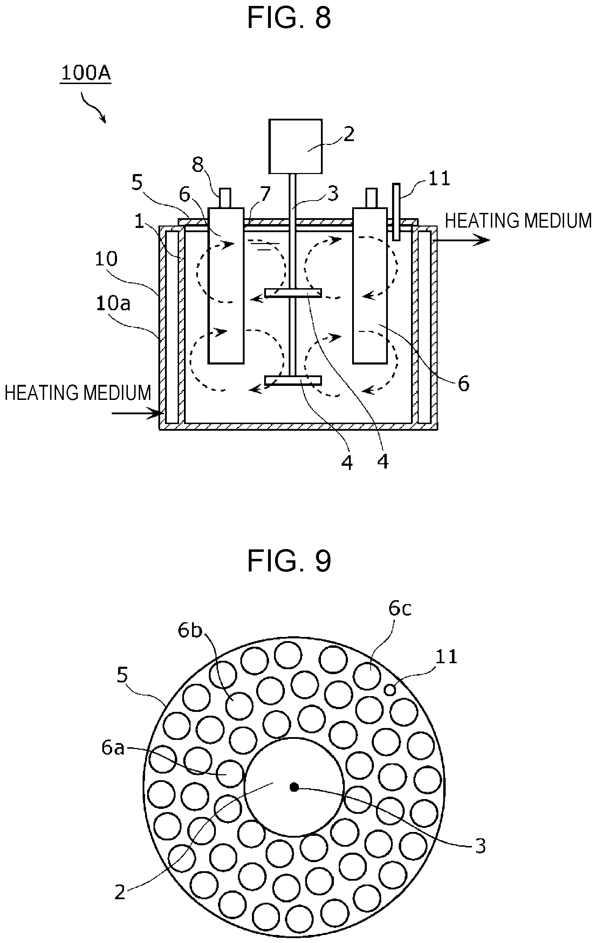

Fig. 8 illustrates one example of a configuration of a stirrer 2A of a food processing apparatus 100A according to Modification 1.

As illustrated in Fig. 8, the stirrer 2A includes (for example, two) stirring bodies 4 that are rotated by the rotary shaft 3 that is common therebetween. Specifically, the stirrer 2A may include the stirring bodies 4 at different positions at the rotary shaft 3 in the axial direction. By disposing the stirring bodies 4 at the common rotary shaft 3, it is possible to improve the performance of stirring the reactant by the stirrer 2A. As a result, the flow (schematically indicated by the dotted lines with arrows in Fig. 8) of the reactant moves toward the temperature adjuster 10, and flows that move from the temperature adjuster 10 toward the center and that circulate in the reaction tank 1 are each formed by the stirring bodies 4. It is thus possible to effectively adjust the temperature of the reactant. In other words, by increasing the number of the stirring bodies 4 that are disposed at the single rotary shaft 3, it is possible to improve reactivity of the reactant with respect to the photocatalyst and the performance of temperature control of the reactant.

### (Modification 2)

Fig. 9 illustrates one example of an installation configuration of catalytic reactors 6 of a food processing apparatus according to Modification 2.

As illustrated in Fig. 9, the catalytic reactors 6 may include first catalytic reactors 6a that are disposed to surround the rotary shaft 3 of the stirrer 2 and second catalytic reactors 6b that are disposed to surround the outer side of the first catalytic reactors 6a. The catalytic reactors 6 may include the first catalytic reactors 6a that are located in a first region and the second catalytic reactors 6b that are located in a second region. The first region may be a region between a region whose distance from the rotary shaft 3 is a first distance and a region whose distance from the rotary shaft 3 is a second distance. The second region may be a region between a region whose distance from the rotary shaft 3 is a third distance and a region whose distance from the rotary shaft 3 is a fourth distance. The first distance may be smaller than the second distance, the second distance may be smaller than the third distance, and the third distance may be smaller than the fourth distance. In the present modification, an arrangement configuration in which the catalytic reactors 6 include third catalytic reactors 6c that are disposed to surround the further outer side of the second catalytic reactors 6b is presented as an example. The first catalytic reactors 6a are disposed in a state of being spaced from each other, the second catalytic reactors 6b are disposed in a state of being spaced from each other, and the third catalytic reactors 6c are disposed in a state of being spaced from each other.

In the circumferential direction of an imaginary circle around the rotary shaft 3, the first catalytic reactors 6a are disposed at positions that differ from the positions of the second catalytic reactors 6b. In other words, in the aforementioned circumferential direction, the first catalytic reactors 6a are each disposed at a position between, among the second catalytic reactors 6b, mutually adjacent two second catalytic reactors 6b. In the circumferential direction of the imaginary circle around the rotary shaft 3, the second catalytic reactors 6b are disposed at positions that differ from the positions of the third catalytic reactors 6c. In other words, in the aforementioned circumferential direction, the second catalytic reactors 6b are each disposed at a position between, among the third catalytic reactors 6c, two mutually adjacent third catalytic reactors 6c.

Consequently, the reactant that has passed among the first catalytic reactors 6a easily comes into contact with the second catalytic reactors 6b disposed on the outer side thereof. The reactant that has passed among the second catalytic reactors 6b easily comes into contact with the third catalytic reactors 6c disposed on the outer side thereof. Reversely, the reactant that has passed among the third catalytic reactors 6c easily comes into contact with the second catalytic reactors 6b disposed on the inner side thereof. The reactant that has passed among the second catalytic reactors 6b easily comes into contact with the first catalytic reactors 6a disposed on the inner side thereof. It is thus possible to improve the reactivity of the reactant with respect to the catalytic reactors 6.

### (Modification 3)

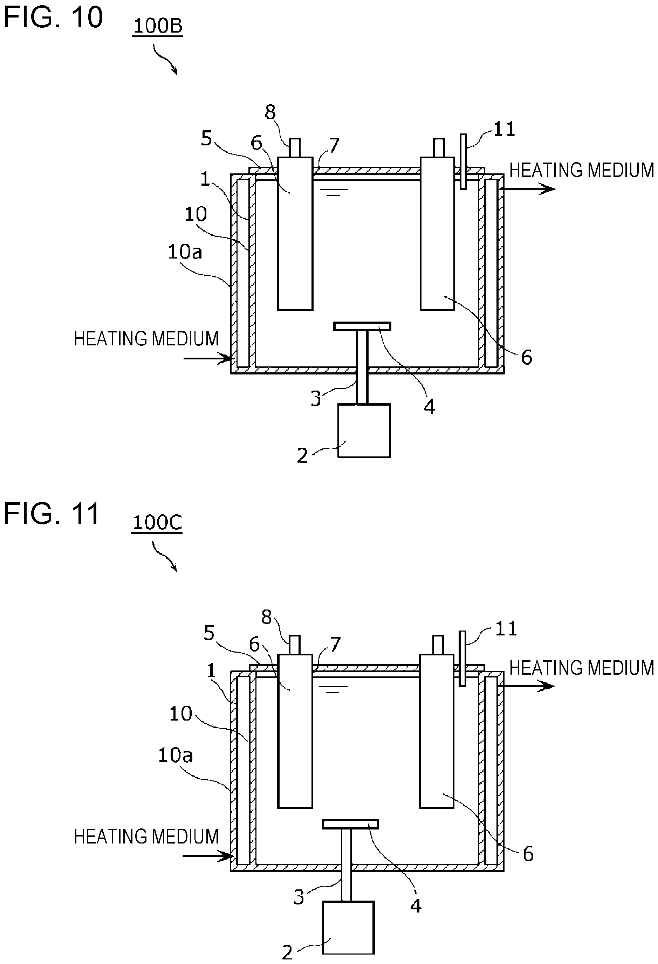

Fig. 10 illustrates one example of a food processing apparatus 100B according to Modification 3.

In the food processing apparatus 100 according to the aforementioned embodiment, the rotary shaft 3 of the stirrer 2 is configured to be disposed to pass through the lid 5 provided at an upper portion of the reaction tank 1. The rotary shaft 3 is, however, not limited thereto. For example, as in the food processing apparatus 100B illustrated in Fig. 10, the stirrer 2 may be configured to be disposed with the rotary shaft 3 of the stirrer 2 passing through the bottom 1a of the reaction tank 1.

### (Modification 4)

Fig. 11 illustrates one example of a food processing apparatus 100C according to Modification 4.

In the food processing apparatus 100 according to the aforementioned embodiment, the rotary shaft 3 of the stirrer 2 is disposed to correspond to the center axis of the cylinder of the reaction tank 1. The stirrer 2 is, however, not limited thereto. For example, as in the food processing apparatus 100C illustrated in Fig. 11, the stirrer 2 may be disposed such that the rotary shaft 3 of the stirrer 2 is at a position shifted from the center axis of the cylinder of the reaction tank 1. While Fig. 11 illustrates, as an example, a configuration in which the rotary shaft 3 of the stirrer 2 passes through the bottom 1a of the reaction tank 1 as in Modification 3, a configuration in which, as in the embodiment, the rotary shaft 3 passes through the lid 5 of the reaction tank 1 may employed.

### (Modification 5)

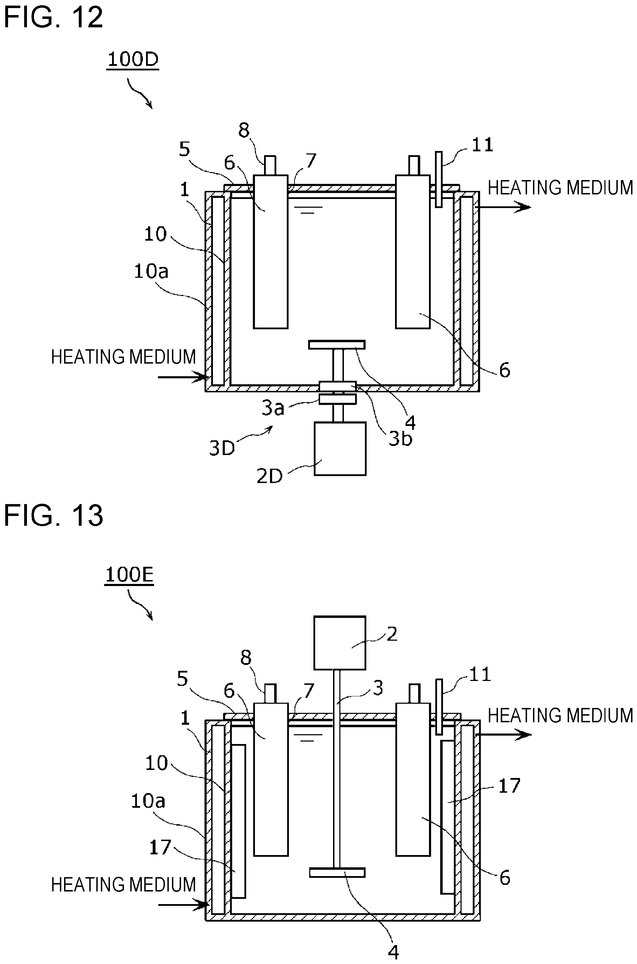

Fig. 12 illustrates one example of a food processing apparatus 100D according to Modification 5.

In the food processing apparatus 100B according to Modification 3 mentioned above, the rotary shaft 3 of the stirrer 2 is configured to be disposed to pass through the bottom 1a of the reaction tank 1. The rotary shaft 3 of the stirrer 2 is, however, not limited thereto. For example, as in the food processing apparatus 100D illustrated in Fig. 12, a rotary shaft 3D of a stirrer 2D may be configured to include an outside rotary shaft 3a that is disposed on the outer side of the reaction tank 1 and an inside rotary shaft 3b that is disposed on the inner side of the reaction tank 1 and that is magnetically coupled to the outside rotary shaft 3. The outside rotary shaft 3a is connected to a motor, not illustrated, of the stirrer 2D and is rotated by receiving a rotational force from the motor. The inside rotary shaft 3b receives a magnetic action of the outside rotary shaft 3a and rotates in accordance with the rotation of the outside rotary shaft 3a. The inside rotary shaft 3b is provided with the stirring body 4, and the inside rotary shaft 3b stirs the reactant in the reaction tank 1 by rotating. As described above, the outside rotary shaft 3a and the inside rotary shaft 3b constitute a magnet coupling, and the stirrer 2D can stir the reactant in the reaction tank 1 without passing through the reaction tank 1. Therefore, there is no need to provide the bottom 1a of the reaction tank 1 with a seal structure for preventing the reactant from leaking to the outside of the reaction tank 1.

### (Modification 6)

Fig. 13 illustrates one example of a food processing apparatus 100E according to Modification 6.

The food processing apparatus 100E according to Modification 6 differs from the food processing apparatus 100 according to the embodiment illustrated in Fig. 1 in that stirring plates 17 are further included in the food processing apparatus 100.

Each of the stirring plates 17 protrudes from the inner wall surface of the reaction tank 1 toward the inner side of the reaction tank 1 and is disposed along the rotary shaft 3. Each of the stirring plates 17 is, for example, an elongated plate-shaped member and is disposed in an orientation in which a short direction is along a direction toward the rotary shaft 3 and a longitudinal direction is along the rotary shaft 3. The stirring plates 17 include, for example, six stirring plates and, in the circumferential direction of an imaginary circle around the rotary shaft 3, are disposed at positions that differ from the positions of the catalytic reactors 6. In other words, in the aforementioned circumferential direction, the stirring plates 17 are each disposed at a position between, among the catalytic reactors 6, two mutually adjacent catalytic reactors 6. The stirring plates 17 are, for example, members made of metal

Consequently, the flow of the reactant generated by the stirrer 2 and moving along the inner surface of the temperature adjuster 10 comes into contact with the stirring plates 17 and can flow upward more along the inner surface of the temperature adjuster 10. Since the stirring plates 17 are fixed to the inner wall surface of the reaction tank 1, the heat caused by the temperature adjuster 10 is conducted to the stirring plates 17. The stirring plates 17 thus can also function as a heat conductor (in other words, a fin) that accelerates heat exchange between the reactant in the reaction tank 1 and the heating medium of the temperature adjuster 10. It is thus possible to obtain an effect of more effectively adjusting the temperature of the reactant.

While food processing apparatuses according to one aspect or aspects of the present disclosure have been described above on the basis of an embodiment, the present disclosure is not limited to this embodiment. As long as not departing from the gist of the present disclosure, embodiments that are obtained by applying various modifications conceived by those skilled in the art to the present embodiment and embodiments that are constituted by a combination of components in different embodiments may be also included in the scope of the one aspect or aspects of the present disclosure.

### Industrial Applicability

One aspect of the present disclosure is usable in, for example, a food processing apparatus in which a photocatalyst that reforms a raw material of a food is used.

### Reference Signs List

1 reaction tank
2 stirrer
3 rotary shaft
4 stirring body
5 lid
6 catalytic reactor
7 reaction tube
7a glass substrate
7b thin film
8 light source
10 temperature adjuster
10a outer wall
11 temperature detector
13 pressing portion
14 movable pressing portion
15 pressing screw
16 bottom-side fixing member
17 stirring plate
100, 100A to 100E food processing apparatus

## Claims

1. A food processing apparatus comprising:
a reaction tank that has a first space for storing a liquid reactant that is to be used for a food;
a stirrer that includes a first stirring body that stirs the reactant in the reaction tank by rotating;
catalytic reactors, the catalytic reactors each including a reaction tube and a light source disposed in the reaction tube, the reaction tube having an outer surface provided with a photocatalyst, the reaction tube allowing light that is emitted from the light source to pass through the reaction tube; and
a temperature adjuster that adjusts a temperature of the reactant in the reaction tank,
wherein the catalytic reactors are disposed around a first rotary shaft of the first stirring body to be spaced from each other, and
wherein the temperature adjuster surrounds the catalytic reactors.

2. The food processing apparatus according to claim 1,
wherein the temperature adjuster includes an outer wall that surrounds the reaction tank, and a heating medium that circulates in a second space between the reaction tank and the outer wall.

3. The food processing apparatus according to claim 2,
wherein the heating medium is a refrigerant that cools the reactant.

4. The food processing apparatus according to any one of claims 1 to 3, further comprising:
a temperature detector that is disposed in the reaction tank and detects a temperature of the reactant,
wherein the temperature adjuster operates based on the temperature detected by the temperature detector to thereby adjust the temperature of the reactant.

5. The food processing apparatus according to any one of claims 1 to 4,
wherein the first stirring body includes at least one of a propeller blade, a disk turbine blade, an inclined paddle blade, and a centrifugal stirring body.

6. The food processing apparatus according to any one of claims 1 to 5,
wherein the stirrer includes a second stirring body, and the first rotary shaft and the second stirring body have an identical rotational axis.

7. The food processing apparatus according to any one of claims 1 to 6, further comprising:
a stirring plate that protrudes from an inner wall surface of the reaction tank toward an inner side of the reaction tank and that is disposed along the first rotary shaft.

8. The food processing apparatus according to any one of claims 1 to 7,
wherein the catalytic reactors include first catalytic reactors that are located in a first region and second catalytic reactors that are located in a second region,
wherein the first region is a region between a region whose distance from the first rotary shaft is a first distance and a region whose distance from the first rotary shaft is a second distance,
wherein the second region is a region between a region whose distance from the first rotary shaft is a third distance and a region whose distance from the first rotary shaft is a fourth distance, and
wherein the first distance is smaller than the second distance, the second distance is smaller than the third distance, and the third distance is smaller than the fourth distance.

9. The food processing apparatus according to any one of claims 1 to 8,
wherein the reaction tube includes a bottomed cylindrical glass substrate and a thin film of a photocatalyst provided on an outer surface of the glass substrate, and
wherein the glass substrate is disposed in an orientation in which a cylinder axis direction of the glass substrate is along the first rotary shaft.

10. The food processing apparatus according to any one of claims 1 to 9, further comprising;
a lid that covers an upper portion of the reaction tank,
wherein the lid includes a lid-side fixing member that fixes the catalytic reactors.

11. The food processing apparatus according to any one of claims 1 to 10, further comprising;
a bottom-side fixing member that is disposed at a bottom of the reaction tank and fixes the catalytic reactors to the bottom.

12. A light-source-equipped reaction tube that is to be used for a catalytic reactor of the food processing apparatus according to any one of claims 1 to 11, the light-source-equipped reaction tube comprising:
a reaction tube that has an outer surface provided with a photocatalyst and that allows light to pass through the reaction tube, and
a light source that emits light from an inner side of the reaction tube.
